# EUROPÄISCHE PATENTSCHRIFT

(11) **EP 0 548 621 B1**
(45) Veröffentlichungstag und Bekanntmachung des Hinweises auf die Patenterteilung: **17.08.1994**
(21) Anmeldenummer: 92120629.8
(22) Anmeldetag: 03.12.1992
(51) Int. Cl.: A61K 7/06, A61K 7/09

(54) **Verfahren zum Fixieren von dauerwellen und Fixiermittel für Dauerwellen mit Peroxidasen**
Process for fixing permanent wave and fixative using peroxidases
Procédé de fixation et compositions fixant les permanentes utilisant des peroxydases

(30) Priorität: 20.12.1991 DE 4142277; 04.04.1992 DE 4211371
(43) Veröffentlichungstag der Anmeldung: 30.06.1993
(73) Patentinhaber: GOLDWELL GmbH, D-64297 Darmstadt (DE)
(72) Erfinder: Dornbusch, Klaus, Dr., W-8771 Erlenbach (DE); Olt, Andrea, W-6100 Darmstadt (DE); Tennigkeit, Jürgen, Dr., W-6104 Seeheim-Jugenheim (DE); Rose, Burkhard, W-6100 Darmstadt 13 (DE); Schneider, Jörg, W-6103 Griesheim (DE)

(56) Entgegenhaltungen:
- EP-A- 0 310 675
- EP-A- 0 502 304

## Beschreibung

Die vorliegende Erfindung betrifft ein Verfahren zum Fixieren von Dauerwellen und ein Fixiermittel für Dauerwellen, das eine haarschonende Behandlung unter Vermeidung einer Schädigung gestattet.

Bekanntlich erfolgt die Dauerverformung menschlicher Haare, das sogenannte Dauerwellen, in zwei Stufen:
Die reduktive Spaltung der Cystin-Disulfidbrücken des Haares durch Einwirkung eines Reduktionsmittels, und die anschließende Fixierung durch Aufbringung eines Oxidationsmittels, wodurch die Cystin-Disulfidbrücken wiederhergestellt werden.

Das klassische Reduktionsmittel ist, wie bereits aus den Pionierpatenten DE-PS 948 186 und 972 424 hervorgeht, dabei die Thioglycolsäure, z.B. als Ammonium- oder Monoethanolaminsalz, die in den letzten Jahren durch Glycerinmonothioglykolat teilweise ersetzt wurde; jedoch können auch Thiomilchsäure und deren Ester sowie anorganische Sulfite eingesetzt werden.

Die Fixierung des verformten Haares wird durch Einwirkung von Oxidationsmitteln, insbesondere Wasserstoffperoxid, vorgenommen, wobei in der Regel Konzentrationen von etwa 2,5 bis 5 Gewichtsprozent zum Einsatz gelangen.

Die Einwirkung derart hoher Oxidationsmittel-Konzentrationen kann, insbesondere auch bei zusätzlich oxidativ in alkalischem Medium gefärbtem oder gebleichtem Haar, eine Haarschädigung hervorrufen.

Die Erfindung geht daher von der Aufgabenstellung aus, ein Fixiermittel und ein Verfahren zum Fixieren von Dauerwellen zur Verfügung zu stellen, das eine schonende Haarbehandlung gewährleistet.

Die Lösung dieser Aufgabe besteht darin, ein Mittel zur Fixierung anzuwenden, das ein Oxidationsmittel in einer wesentlich geringeren als der üblichen Konzentration, nämlich 0,01 bis 1 Gewichtsprozent, vorzugsweise 0,02 bis 0,1 Gewichtsprozent, berechnet als H₂O₂ auf die Gesamtzusammensetzung, und zusätzlich eine katalasefreie Peroxidase enthält.

Durch die erfindungsgemäß erforderliche Abwesenheit der Katalase-Aktivität ist die Peroxidase bei der Anwendung geeignet, die Fixierung der Dauerwelle auch durch geringe Mengen Peroxid zu katalysieren.

Um die Wirkung des erfindungsgemäßen Mittels noch zu verbessern, ist es zweckmäßig, den das Enzym enthaltenden Zusammensetzungen noch ein Benetzungsmittel zuzusetzen, um die Aufnahmefähigkeit der Haare zu verbessern.

Ein geeignetes Benetzungsmittel ist dabei ein nichtionisches Tensid, das in einer Menge von etwa 0,1 bis etwa 4,0 Gewichtsprozent zugesetzt werden kann.

Ein bevorzugtes nichtionisches Tensid ist insbesondere ein Ethylenoxid/Propylenoxid-Copolykondensat der allgemeinen Formel

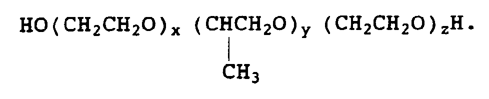

Ein besonders geeignetes Copolykondensat der obengenannten Formel ist ein solches, wo die Durchschnittswerte für x, y und z jeweils bei 98,67 und 98 liegen.
Ein derartiges Produkt ist unter der Bezeichnung "Poloxamer 407" bekannt und unter dem Handelsnamen "Genapol^{R} PF70" oder "Pluronic F127" erhältlich.

Geeignet sind jedoch auch andere "Poloxamer"-Typen der obengenannten allgemeinen Formel, wo die Mittelwerte für x und z zwischen 2 und 128 und diejenigen für y zwischen 16 und 67 liegen.

Geeignete Poloxamere sind im CTFA International Cosmetic Ingredient Dictionary, Fourth Edition (1991), S.447 bis 451, beschrieben.

Wie bereits ausgeführt, werden diese Tenside vorzugsweise in einer Konzentration zwischen 0,1 und 4 Gewichtsprozent, insbesondere 0,1 bis 2,5, ganz bevorzugt 0,5 bis 1,5 %, eingesetzt.

Weitere geeignete Tenside sind Alkylglucoside der allgemeinen Formel RO(OR¹)ₜZₓ, worin R eine Alkylgruppe mit 8 bis 18 Kohlenstoffatomen, R¹ einen Ethylen- oder Propylenrest, Z ein reduzierendes Saccharid mit 5 bis 6 Kohlenstoffatomen, t null bis 10 und x eins bis 5 bedeuten.

Auch diese Tenside sind geeignet, eine genügende Benetzbarkeit des Haares zu erreichen, ohne die Stabilität der Peroxidase zu beeinträchtigen.

Auch C₁₀-C₁₈-Alkylpolyglycolether, beispielsweise mit 5 bis 25 Ethylenoxid-Einheiten/Molekül, können, gegebenenfalls im Gemisch mit anderen Tensiden, eingesetzt werden. Solche Produkte sind beispielsweise unter dem Handelsnamen "Genapol^{R}" bekannt.

Die erfindungsgemäß zum Einsatz gelangende Peroxidase kann pflanzlichen Ursprungs oder aus Bakterien gewonnen worden sein. Maßgeblich ist, daß sie im wesentlichen keine Katalase-Aktivität enthält.

Geeignete pflanzliche Herkunftsquellen sind beispielsweise Meerrettich, Sojabohnen, Feigen und Erdnüsse; geeignete Bakterienstämme zur Herstellung von Peroxidasen sind beispielsweise Acetobacter, Staphylococcen und Lactobacillen; auch aus Bäckerhefe gewonnene katalasefreie Peroxidasen sind geeignet.

Prinzipiell sind alle in der Klasse 1.11 der "Classification of the International Union of Biochemistry on the Nomenclature and Classification of Enzymes (1964)" aufgeführten Peroxidasen im Rahmen der vorliegenden Erfindung geeignet.

Besonders bevorzugt ist hierbei das unter der Nummer 1.11.1.7. klassifizierte Peroxidase-Enzym, jedoch können beispielsweise auch NAD-Peroxidase (1.11.1.1.), NADP-Peroxidase (1.11.1.2.), Fettsäure-Peroxidase (1.11.1.3.), Cytochrom-Peroxidase (1.11.1.5.) und Glutathion-Peroxidase (1.11.1.9.) verwendet werden.

Die bevorzugte Enzymaktivität bei der Anwendung beträgt von etwa 3 bis etwa 500 Peroxidase-Einheiten (U) pro 100 g der Enzymlösung. Der besonders bevorzugte Wert liegt bei etwa 10 bis etwa 150 U/100 g, insbesondere 15 bis 50 U/100 g Enzymzusammensetzung.

Berechnet auf reines Enzym, liegt die Aktivität bei mehr als 200 U/mg, insbesondere mehr als 250 U/mg.

Mit "katalasefrei" werden solche Enzymzusammensetzungen bezeichnet, deren Katalase-Aktivität weniger als 0,7 %, vorzugsweise unter 0,5 % der Gesamt-Peroxidase-Aktivität, beträgt.

Die Bestimmungsmethode der Peroxidase-Aktivität ist seit langem bekannt und in diversen Veröffentlichungen beschrieben.

Das erfindungsgemäße Verfahren zur Dauervellfixierung wird vorzugsweise so durchgeführt, daß auf das mit dem Verformungsmittel behandelte, gespülte Haar zunächst die Enzymzusammensetzung aufgebracht und anschließend mit der niedrig konzentrierten Oxidationsmittel-Zusammensetzung behandelt wird.

Alternativ dazu kann auch unmittelbar vor der Fixierung die Enzymzusammensetzung mit der Peroxidzusammensetzung vereinigt und diese sofort auf das verformte Haar aufgebracht werden.

Es ist auch möglich, Enzym und Oxidationsmittel in einer Zusammensetzung zu lagern. Voraussetzung hierfür ist, daß eine der beiden Substanzen bis zur Einwirkung auf das Haar immobilisiert wird, was beispielsweise durch Einkapselung des Oxidationsmittels, insbesondere eines Peroxids, oder des Enzyms, geschieht, so daß dieses erst bei der Aufbringung auf das Haar, entweder durch mechanisches Aufbrechen der Kapsel oder chemisch (beispielsweise durch Variation des pH-Wertes) bedingte Freigabe freigesetzt wird.

Falls es sich bei dem Oxidationsmittel um ein bei der Anwendung aufzulösendes Pulver oder eine Täblette handelt, kann das ebenfalls pulverförmige Peroxidase-Enzym auch diesem Pulver bzw. der Tablette zugesetzt werden.

Im Rahmen des erfindungsgemäßen Verfahrens können die an sich bekannten Fixierlösungen zum Einsatz gelangen, mit Ausnahme der Tatsache selbstverständlich, daß die Konzentration des Oxidationsmittels, beispielsweise Wasserstoffperoxid, wesentlich herabgesetzt ist.
Die Konzentration des Peroxids liegt dabei zwischen etwa 0,01 und etwa 1, vorzugsweise 0,02 bis 0,5, insbesondere 0,02 bis etwa 0,1 Gew.-% der Fixiermittel-Zusammensetzung, berechnet als Wasserstoffperoxid.
Wird ein anderes Oxidationsmittel, beispielsweise Natriumbromat oder Natriumperborat, eingesetzt, muß die Konzentration entsprechend umgerechnet werden.

Die DE-A 1 048 389 beschreibt ein Verfahren zur Haarumformung, das dadurch gekennzeichnet ist, daß das Haar mit einer alkalischen Lösung einer organischen Thioverbindung R₁-CO-S-R₂ behandelt wird, der ein Esterase und Oxidase enthaltendes Leberpräparat zugesetzt ist. Der Zusatz der Esterasen soll eine Spaltung der CO-S-Bindungen bewirken. Mit der erfindungsgemäßen Wirkung der Peroxidase hat dies nichts gemeinsam, zumal Leberextrakte üblicherweise auch beträchtliche Katalase-Aktivität aufweisen.

Aus der EP-A 310 675 sind bereits Haarpflegemittel-Zusammensetzungen bekannt, die als aktiven Bestandteil eine Oxidase der Gruppe Pyranose-Oxidase, Glucose-Oxidase, Glycerin-Oxidase, Milchsäure-Oxidase, Brenztraubensäure-Oxidase und Uricase enthalten, für die Sauerstoff einen Akzeptor darstellt.
Durch die Verwendung dieser Enzyme soll insbesondere der Einsatz von Peroxiden wie Wasserstoffperoxid in Fixiermitteln für Dauerwellen und Haarfärbemitteln auf Basis von Oxidationsfarbstoff-Vorprodukten überflüssig gemacht werden.

Die in den Beispielen 2 sowie 11 bis 13 der EP-A 310 675 verwendeten Fixierlösungen enthalten demzufolge auch kein Wasserstoffperoxid, da diese Veröffentlichung, wie oben dargestellt, von einer anderen Aufgabenstellung ausgeht und deshalb mit der vorliegenden Erfindung in keinem näheren Zusammenhang steht.

Es wurde gefunden, daß die mit dem Verfahren nach dem älteren Patent unter Einsatz des entsprechenden Mittels erzielte Fixierung noch verbessert werden kann, wenn im Anschluß an diese, mit oder ohne zwischenzeitliche Spülung, eine säurehaltige Zusammensetzung auf das Haar aufgebracht wird, die einen pH-Wert von 1 bis etwa 6,5, insbesondere etwa 1,5 bis etwa 6, vorzugsweise zwischen 2 und 5,5, aufweist.

Bei über 5,5 liegenden pH-Werten ist dabei in der Regel der Zusatz eines Puffers erforderlich, was aufgrund der dann notwendigen Anwesenheit von Salzen zur teilweisen Ausfällung dieser Salze auf dem Haar führen kann, was an sich unerwünscht ist.

Im Prinzip ist jede Säure geeignet, in der erfindungsgemäßen sauren Spülzusammensetzung Verwendung zu finden.

Neben den üblichen Mineralsäuren wie Salzsäure, Schwefelsäure, Salpetersäure und Phosphorsäure, sind insbesondere organische Säuren geeignet. Bevorzugt werden hierbei Citronensäure, Glyoxylsäure, Maleinsäure, Fumarsäure, Essigsäure, Propionsäure, Hydroxypropionsäure, Weinsäure, Apfelsäure, Äpfelsäure, Dicarbonsäuren wie Oxalsäure, Bernsteinsäure, Glutarsäure, Adipinsäure, etc., saure Salze derselben und Kombinationen der genannten Säuren untereinander und mit anderen Säuren bzw. sauren Salzen.

Die Aufbringung dieser sauren "Nachfixierung" erfolgt unmittelbar im Anschluß an die Behandlung nach dem Hauptpatent mit einer Wasserstoffperoxid in geringer Konzentration sowie eine katalasefreie Peroxidase enthaltenden Zusammensetzung.

Nach etwa 3- bis etwa 10minütiger Einwirkung der sauren Nachfixierung wird dann mit Wasser gespült.

Obwohl es an sich zur Erreichung der angestrebten Wirkung ausreichend ist, eine einfache wäßrige Lösung der Säure, gegebenenfalls unter Zusatz entsprechender Puffersubstanzen, zu verwenden, ist es doch zweckmäßig, dieser weitere Hilfsstoffe zuzusetzen. Solche sind beispielsweise, neben Parfum, haarkonditionierende Substanzen wie Eiweißhydrolysate oder kationische Polymere, oberflächenaktive Substanzen, sofern eine Schaumwirkung erwünscht ist, gegebenenfalls Lösungsvermittler für die Parfumöle, erwünschtenfalls Farbstoffe etc.

Bei der einfachsten Ausführungsform der erfindungsgemäßen sauren Fixiermittel-Zusammensetzung handelt es sich um eine wäßrige Lösung, jedoch kann sie beispielsweise auch als Emulsion, Gel oder Aerosolschaum zum Einsatz gelangen.

Im folgenden wird anhand von Beispielen die Erfindung näher illustriert.

Sämtliche Behandlungen wurden an natürlichen Haarsträhnen durchgeführt, die durch Behandlung mit einer handelsüblichen Dauerwell-Zusammensetzung verformt und anschließend ausgespült wurden.

### Beispiel 1

Es wurde eine wäßrige Fixierlösung, enthaltend
- 0,1 g: Wasserstoffperoxid,
- 16 U/100 g: Peroxidase (1.11.1.7),
- 1,0 g: Poloxamer 407,

auf 100 g Wasser hergestellt.

Die Lösung war mit KH₂PO₄/K₂HPO₄-Puffer (0,1 M) auf pH 7,0 eingestellt.

Nach zehnminütiger Einwirkungszeit wurde gespült.

Dehnungsmessungen ergaben, daß die mit dieser Lösung fixierten Haarsträhnen gegenüber mit einem üblichen Fixiermittel mit einer Wasserstoffperoxid-Konzentration von 3,5 Gewichtsprozent behandelten Strähnen eine wesentlich bessere Elastizität aufweisen, was auf eine minimale Haarschädigung der erfindungsgemäßen im Vergleich zur konventionellen Fixierung schließen läßt.

### Beispiel 2

Mit einer wäßrigen Fixierlösung, bestehend aus
- 0,2 g: Wasserstoffperoxid,
- 160 U/100 g: Peroxidase (1.11.1.7)
- 1,0 g: C₉-C₁₁-Alkylpolyglycosid (Kondensationsgrad x = 1,5),

eingestellt mit Pufferlösung auf pH 7, wurden gewellte Haarsträhnen 10 Minuten fixiert und anschließend mit einer an sich bekannten, schwach sauren Nachbehandlungslösung behandelt.

Es wurde die gleiche Elastizität des so behandelten Haares wie bei einer unbehandelten Haarsträhne trotz eines sehr guten Wellergebnisses gemessen; d.h., das gewellte Haar wies keine Schädigung auf.

### Beispiel 3

Eine mit Wellmittel behandelte und gespülte Haarsträhne wurde zunächst mit einer mit 0,1M KH₂PO₄/K₂HPO₄-Pufferlösung auf pH 7,0 eingestellten wäßrigen Zusammensetzung mit einem Gehalt an
- 160 U/100 g: Peroxidase (1.11.1.7) und
- 1 Gew.-%: Poloxamer 407

behandelt und 5 Minuten später eine wäßrige Lösung aus
- 0,2 Gew.-%: Wasserstoffperoxid und
- 1 Gew.-%: Poloxamer 338

aufgetragen.

Nach weiterer zehnminütiger Einwirkungszeit wurde mit einem bekannten, schwach sauren Dauerwellnachbehandlungsmittel konditioniert.

Es wurde ein ausgezeichnetes Wellergebnis bei praktisch unstrapaziertem Haar erzielt.

### Beispiel 4

Dauergewelltes Haar wurde mit einer Fixierung nach Beispiel 1 des Hauptpatentes behandelt.

Anschließend wurde mit Wasser gespült und eine 1,5%ige wäßrige Lösung von Citronensäure (pH-Wert: 2,15) aufgebracht und nach fünfminütiger Einwirkung bei Zimmertemperatur mit Wasser gespült.

Reißfestigkeitsmessungen zeigten, daß das mit der "sauren Nachspülung" behandelte Haar wesentlich strapazierfähiger ist als das lediglich mit der Fixierung nach Beispiel 1 des Hauptpatents behandelte Haar.

### Beispiel 5

Es wurde wie in Beispiel 1 verfahren, die Citronensäure-Lösung jedoch durch eine Lösung von je 1,5 Gew.-% Glyoxylsäure und Maleinsäure ersetzt (pH-Wert: 1,10).

Es wurde eine deutlich verbesserte Reißfestigkeit des erfindungsgemäß behandelten Haares gemessen.

Die erfindungsgemäßen Fixiermittel können selbstverständlich die üblichen Zusätze und Hilfsmittel wie Konditioner, Parfums, Stabilisatoren, etc., enthalten. Diese sind dem Fachmann bekannt und in den einschlägigen Handbüchern, z. B. K. Schrader, "Grundlagen und Rezepturen der Kosmetika", 2. Auflage (Hüthig Buch Verlag, 1989), S. 823 - 840, beispielhaft beschrieben.

## Patentansprüche

1. Verfahren zur Fixierung von Dauerwellen, dadurch gekennzeichnet, daß auf das mit einem mindestens eine Thioverbindung enthaltenden Wellmittel behandelte, gespülte Haar eine Zusammensetzung aufgebracht wird, die eine katalasefreie Peroxidase enthält, und gleichzeitig oder anschließend mit einer Zusammensetzung behandelt wird, die 0,01 bis 1 Gewichtsprozent eines Oxidationsmittels, berechnet als Wasserstoffperoxid, enthält.

2. Verfahren nach Anspruch 1, dadurch gekennzeichnet, daß als Oxidationsmittel Wasserstoffperoxid in einer Menge von 0,02 bis 0,5 Gewichtsprozent, berechnet auf die Gesamtzusammensetzung, verwendet wird.

3. Mittel zur Fixierung von Dauerwellen, enthaltend 0,01 bis 1 Gewichtsprozent, berechnet auf die Gesamtzusammensetzung, als Wasserstoffperoxid eines Oxidationsmittels, dadurch gekennzeichnet, daß es in räumlich getrennter Zusammensetzung davon eine katalasefreie Peroxidase enthält.

4. Mittel nach Anspruch 3, gekennzeichnet durch einen Gehalt an 0,02 bis 0,1 Gewichtsprozent Wasserstoffperoxid, berechnet auf die Gesamtzusammensetzung.

5. Mittel nach Anspruch 3 oder 4, gekennzeichnet durch einen Gehalt an 3 bis 500 Peroxidase-Einheiten/100 g der Gesamtzusammensetzung.

6. Mittel nach einem der Ansprüche 3 bis 5, dadurch gekennzeichnet, daß es etwa 0,1 bis etwa 4,0 Gewichtsprozent mindestens eines nichtionischen Tensids enthält.

7. Mittel nach Anspruch 6, dadurch gekennzeichnet, daß es als nichtionisches Tensid ein Alkylglycosid der allgemeinen Formel
RO(OR¹)ₜZₓ,
worin R eine Alkylgruppe mit 8 bis 18 Kohlenstoffatomen, R¹ einen Ethylen- oder Propylenrest, z ein reduzierendes Saccharid mit 5 bis 6 Kohlenstoffatomen, t null bis 10 und x eins bis 5 bedeuten, enthält.

8. Mittel nach Anspruch 6, dadurch gekennzeichnet, daß es als nichtionisches Tensid ein Ethylenoxid/Propylenoxid-Copolykondensat enthält.

9. Mittel nach Anspruch 8, dadurch gekennzeichnet, daß es als Ethylenoxid/Propylenoxid-Copolykondensat ein solches der allgemeinen Formel 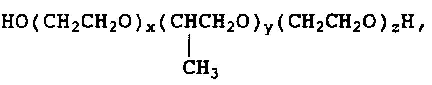 wobei die Durchschnittswerte für x und z jeweils zwischen 2 und 128 und für y zwischen 16 und 67 liegen, enthält.

10. Mittel nach Anspruch 9, dadurch gekennzeichnet, daß die Durchschnittswerte für x und z bei jeweils 98 und für y bei 67 liegen.

11. Verfahren zur Fixierung von Dauerwellen nach Anspruch 1, dadurch gekennzeichnet, daß auf das Haar anschließend eine Säure-Lösung mit einem pH-Wert zwischen 1 und 6,5 aufgebracht wird.

12. Mittel zur Durchführung des Verfahrens nach Anspruch 11, dadurch gekennzeichnet, daß es aus einer Säure-Lösung mit einem pH-Wert zwischen 1 und 6,5 besteht.

13. Mittel nach Anspruch 12, dadurch gekennzeichnet, daß es einen pH-Wert zwischen 2 und 5,5 aufweist.

14. Mittel nach Anspruch 12 oder 13, dadurch gekennzeichnet, daß es 0,5 bis 10 Gew.-%, berechnet auf die Gesamtzusammensetzung, Citronensäure enthält.

15. Mittel nach Anspruch 12 oder 13, dadurch gekennzeichnet, daß es 0,5 bis 10 Gew.-% Glyoxylsäure und/oder Maleinsäure enthält.

## Claims

1. Process for neutralizing of permanent waves, characterized in that onto hair having been treated with a waving composition containing at least one thio compound and rinsed afterwards, a composition is applied containing a peroxidase enzyme being substantially free from catalase activity, and at the same time or afterwards, said hair is treated with a composition containing 0.01 to 1% by weight of an oxidizing agent, calculated as hydrogen peroxide.

2. Process according to claim 1, characterized in that hydrogen peroxide is used as oxidizing agent in an amount of 0.02 to 0.5% by weight, calculated to the total composition.

3. Composition for neutralizing permanent waves containing 0.01 to 1% by weight of an oxidizing agent, calculated to the total composition as hydrogen peroxide, characterized in that it also contains a peroxidase enzyme substantially free of catalase, which is kept separately from said oxidizing composition until application.

4. Composition according to claim 3, characterized in that it contains 0.02 to 0.1% by weight of hydrogen peroxide, calculated to the total composition.

5. Composition according to claim 3 or 4, characterized by a peroxidase activity of 3 to 500 peroxidase units/100g of the total composition.

6. Composition according to one of the claims 3 to 5, characterized in that it contains about 0.1 to about 4.0% by weight of at least one nonionic surfactant.

7. Composition according to claim 6, characterized in that it contains a nonionic alkyl glycoside of the general formula
RO (OR¹)ₜZₓ,
wherein R is a C₈-C₁₈-alkyl group, R¹ is an ethylene or propylene group, Z is a reducing saccharide with 5 to 6 C-atoms, t is zero to ten, and x is 1 to 5.

8. Composition according to claim 6, characterized in that it contains a nonionic ethylene oxide/propylene oxide copoly-condensate.

9. Composition according to claim 8, characterized in that it contains an ethylene oxide/propylene oxide copolycondensate of the general formula 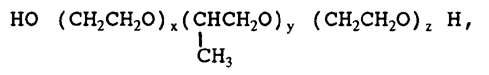 wherein the average numbers for x and z are between 2 and 128, and y is between 16 and 67.

10. Composition according to claim 9, characterized in that the average numbers for x and z are 98 each and for y is 67.

11. Process for neutralizing permanently waved hair according to claim 1 or 2, characterized in that thereafter an acidic solution having a pH value between 1 and 6.5 is applied onto the hair.

12. Composition to perform the process according to claim 11, characterized in that it comprises an acidic solution having a pH value between 1 and 6.5.

13. Composition according to claim 12, characterized in that it has a pH value of between 2 and 5.5.

14. Composition according to claim 12 or 13, characterized that it contains 0.5 to 10% by weight of citric acid, calculated to the total composition.

15. Composition according to claim 12 or 13, characterized in that it contains 0.5 to 10% by weight of glyoxylic acid and (or) maleic acid, calculated to the total composition.

## Revendications

1. Procédé de fixation de permanentes, caractérisé en ce que, sur les cheveux traités par au moins un produit d'ondulation contenant un composé thio et rincés, on applique une composition qui contient une peroxydase exempte de catalase et en ce que simultanément ou ensuite on les traite avec une composition qui contient 0,01 à 1 % en poids d'un oxydant, calculé sous la forme de peroxyde d'hydrogène.

2. Procédé suivant la revendication 1, caractérisé en ce que, comme oxydant, on utilise du peroxyde d'hydrogène en une quantité de 0,02 à 0,5 % en poids, calculée par rapport à la composition globale.

3. Produit de fixation de permanentes, contenant 0,01 à 1 % en poids d'un oxydant, calculé sous la forme de peroxyde d'hydrogène par rapport à la composition globale, caractérisé en ce qu'il contient une peroxydase exempte de catalase dans une composition spatialement séparée de l'oxydant.

4. Produit suivant la revendication 3, caractérisé par une teneur en 0,02 à 0,1 % en poids de peroxyde d'hydrogène, calculé par rapport à la composition globale.

5. Produit suivant l'une des revendications 3 et 4, caractérisé par une teneur en 3 à 500 unités de peroxydase/100 g de la composition globale.

6. Produit suivant l'une des revendications 3 à 5, caractérisé en ce qu'il contient environ 0,1 jusqu'à environ 4,0 % en poids d'au moins un agent tensioactif non ionogène.

7. Produit suivant la revendication 6, caractérisé en ce qu'il contient, comme agent tensioactif non ionogène, un alkylglycoside de la formule générale
RO (OR¹)ₜZₓ,
dans laquelle R représente un groupe alkyle ayant de 8 à 18 atomes de carbone, R¹ représente un radical éthylène ou propylène, Z représente un saccharide réducteur ayant 5 à 6 atomes de carbone, t signifie 0 à 10 et x signifie 1 à 5.

8. Procédé suivant la revendication 6, caractérisé en ce qu'il contient, comme agent tensioactif non ionogène, un produit de copolycondensation d'oxyde d'éthylène/oxyde de propylène.

9. Produit suivant la revendication 8, caractérisé en ce qu'il contient, comme produit de copolycondensation d'oxyde d'éthylène/oxyde de propylène, un produit répondant à la formule générale 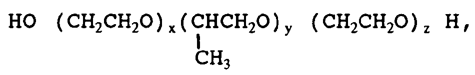 dans laquelle les valeurs moyennes pour x et z sont chacune comprises entre 2 et 128 et pour y entre 16 et 67.

10. Produit suivant la revendication 9, caractérisé en ce que les valeurs moyennes pour x et z sont chaque fois de 98 et pour y de 67.

11. Procédé de fixation de permanentes suivant la revendication 1, caractérisé en ce qu'on applique ensuite sur les cheveux une solution acide ayant une valeur de pH comprise entre 1 et 6,5.

12. Produit pour la mise en oeuvre du procédé suivant la revendication 11, caractérisé en ce qu'il est constitué d'une solution acide ayant une valeur de pH comprise entre 1 et 6,5.

13. Produit suivant la revendication 12, caractérisé en ce qu'il présente une valeur de pH comprise entre 2 et 5,5.

14. Produit suivant l'une des revendications 12 et 13, caractérisé en ce qu'il contient 0,5 à 10 % en poids d'acide citrique, par rapport à la composition globale.

15. Produit suivant l'une des revendications 12 et 13, caractérisé en ce qu'il contient 0,5 à 10 % en poids d'acide glyoxylique et/ou d'acide maléique.
